# EUROPEAN PATENT APPLICATION

(11) **EP 4 674 395 A1**
(43) Date of publication of application: **07.01.2026**
(21) Application number: 24186010.5
(22) Date of filing: 02.07.2024
(51) Int. Cl.: A61F 13/00, A61F 13/06, A61F 13/0206, G06Q 30/016, G06K 19/04

(54) **A MEDICAL DRESSING COMPRISING A MACHINE-READABLE CODE**

(71) Applicant: Mölnlycke Health Care AB, 431 21 Mölndal (SE)
(72) Inventor: FLACH, Niclas, 441 35 ALINGSÅS (SE); JAKOBSSON, Conny, 443 38 LERUM (SE); MELIN, Daniel, 435 30 MÖLNLYCKE (SE)
(74) Representative: Kransell & Wennborg KB

(57) **Abstract**

The present disclosure generally relates to a medical dressing (1) having a first side facing the skin or wound of a patient during use, and an opposite, second side, wherein the medical dressing (1) comprises at least one printed marking (2) being visible on the second side of the medical dressing, wherein the printed marking (2) has a curvilinear shape and in that the printed marking (2) comprises a machine-readable code (3).

## Description

### TECHNICAL FIELD

The present disclosure generally relates to a medical dressing having a first side facing the skin or wound of a patient during use, and an opposite, second side, wherein the medical dressing comprises at least one printed marking being visible on the second side of the medical dressing.

### BACKGROUND

Adhesive medical dressings are frequently used in wound care, both for the purpose of treating wounds and scars and for the purpose of preventing these from occurring in the first place.

An adhesive medical dressing typically comprises an adhesive skin-contact layer arranged to contact the skin or wound of a patient, as well as a top layer, often referred to as a backing layer. In many cases, particularly when the dressing is to be applied to moderate or highly exuding wounds, the dressing further comprises a wound pad arranged between the backing layer and the adhesive skin-contact layer. To protect the dressing from contamination, a release liner may be arranged to cover the adhesive skin-contact layer.

Adhesive medical dressings are typically individually packed in pouches, which keep the dressings sterile prior to use. A plurality of pouches housing the dressings are generally contained and stored in an outer package (a "multi-pack").

The pouch and the outer package may contain a variety of product-identifying information. For example, lot lumbers, batch numbers, and dressing-specific information may be printed on the pouch. Furthermore, instructions relating to a correct use or application of the dressing may be provided in an instruction manual ("Instructions for use", IFU), which comes with the package. Such information may also be printed on the outer package.

However, in many cases, usage- or product-related information (as provided in e.g. an IFU or on the outer package) is not present in the actual care situation and may easily become lost or dislocated. Also, once the dressing has been applied to the wound or skin of a patient, the pouch is typically discarded, and the product- or usage-related information thus becomes lost.

In view of this, it is desirable to print product- or usage- related markings on the actual product, i.e. wound dressing, such that the markings are visibly observable even when the medical dressing has been applied to the skin or wound of a patient.

Product-identifying information as well as usage-specific information may be encoded in machine-readable codes, e.g. QR codes.

US 2011/183712A1 relates to a wound dressing comprising a QR code permanently attached to the outer side of a carrier layer, i.e. top layer of the dressing. The QR code provides a uniform resource locator of an internet site containing usage-specific and/or technical data relating to the wound dressing.

A challenge associated with printing markings, such as machine-readable codes, onto medical dressings is that the manufacturing of medical dressings is generally carried out at high speed and with a plurality of medical dressings arranged in parallel and being cut out simultaneously.

The material web onto which the markings are to be printed needs to be correctly positioned with respect to the other material webs and wound pads to ensure alignment of the printed markings prior to cutting. Many times, the printed markings become arranged slightly skewed or "offset" on the final product. Also, tensions in the assembly web may lead to varying and unpredictable distances between the printed markings.

This problem is particularly prominent when the printed markings are machine-readable codes. Misalignment of square-shaped machine-readable codes yields a visually unpleasant impression, particularly with respect to the peripheral edges and the corners of the dressing and the wound pad.

In view of this, there is room for improvements in the field of printed machine-readable codes.

### SUMMARY

In view of the above-mentioned problems, it is an object of the present disclosure to provide improvements in the field of medical dressings comprising printed machine-readable codes.

According to a first aspect, there is provided a medical dressing having a first side facing the skin or wound of a patient during use, and an opposite, second side, wherein the medical dressing comprises at least one printed marking being visible on the second side of the medical dressing, wherein the printed marking has a curvilinear shape and wherein the printed marking comprises a machine-readable code.

The printed marking comprising the machine-readable code has a curvilinear shape, i.e. a shape which is characterized by smooth and rounded edges rather than sharp angles and straight lines. The printed marking can thus accommodate slight misalignments formed during the printing process without looking skewed or distorted on the actual product.

Unlike square-shaped machine-readable codes, which require precise alignment with respect to the dressing (or pad) edges, curvilinear shapes are more "forgiving". This is particularly beneficial in high-speed manufacturing process, where perfect alignment is a challenge.

The curvilinear shape of the printed marking improves the overall visual appearance of the dressing and overcomes the misalignment issues associated with square-shaped printed codes.

Furthermore, the process for manufacturing the medical dressing, and particularly the printing step, is greatly facilitated since aligning a curvilinear shaped printed marking with the other dressing components is much easier and reduces the likelihood of errors.

Curvilinear printed markings may advantageously be used in various dressing designs, regardless of whether the dressing itself is square-shaped, rectangular or has a shape adapted to fit with a specific body contour.

The printed marking is visible on the second side of the medical dressing. Accordingly, technical information about the dressing or the manufacturing of the dressing, as well as information about the use of the dressing may be comprised in the machine-readable code, and is accessible to the patient, caregiver, or medical personnel upon scanning of the machine-readable code.

In some embodiments, the printed marking consists of a circular or an oval machine-readable code.

Accordingly, the machine-readable code may be designed in a circular or oval format. A circular or oval shaped machine-readable code does not have any sharp corners, which may impair the visual impression when printed on the dressing. Circular and oval machine-readable codes integrate "seamlessly" with the curved edges or corners of medical dressings, particularly when the dressing has a curved or irregular shape.

Furthermore, circular, and oval codes can often be scanned from various angles and orientations, which may reduce the risk of scanning errors.

In alternative embodiments, the at least one printed marking may comprise a functional pattern and a non-functional pattern, wherein the machine-readable code forms the functional pattern, and wherein the non-functional pattern forms the remaining part of the printed marking.

Accordingly, the machine-readable code (the functional pattern) may be square-shaped, but the non-functional pattern extends the printed marking to a curvilinear, e.g. circular or oval, shape.

In this regard, the printed marking masks any misalignment issue of the square-shaped code itself. The non-functional pattern (e.g. dots, lines, squares) does not encode any data, but serves to enhance the visual appearance and mask any alignment issues of the functional pattern.

The functional capabilities and advantages associated with conventional square-shaped machine-readable codes may still be maintained.

The functional pattern may be arranged centrally in the printed marking.

This arrangement facilitates scanning of the machine-readable code (functional pattern).

The non-functional pattern may be arranged to surround the functional pattern. Preferably, the printed marking has a circular shape.

The centrally arranged machine-readable code forms the functional pattern, while the surrounding non-functional pattern gives the printed marking a cohesive, circular appearance.

As mentioned hereinbefore, the machine-readable code may be square-shaped.

Accordingly, the data capacity and error correction benefits of square-shaped machine-readable codes are maintained. A square-shaped machine-readable code is typically associated with ease of scanning by an electronical device, e.g. a mobile phone.

The visual appearance of the non-functional pattern typically resembles the visual appearance of the functional pattern.

Accordingly, a cohesive printed marking is accomplished, yielding an improved visual (and professional) appearance.

The non-functional pattern may comprise a plurality of squares, dots or lines configured to resemble the machine-readable code.

The surface area of the machine-readable code may be from 80 to 500 mm², preferably from 100 to 300 mm².

The machine-readable code must be large enough to secure proper scanning and readability by a scanning device, e.g. a mobile phone. The size of the machine-readable code may vary depending on the size of the medical dressing. Medical dressings come in variety of sizes and a variety of shapes. The surface area of the machine-readable code is preferably proportionate to the size of the medical dressing. For example, a too large machine-readable code on a smaller sized dressing may impair the visual impression.

The medical dressing may comprise at least a backing layer and an adhesive skin-contact layer, wherein the at least one printed marking is printed on the backing layer.

The backing layer is the top layer of the medical dressing. The backing layer has a top side and a bottom side. The top side of the backing layer corresponds to "the second side" of the medical dressing referred to hereinbefore. The machine-readable code may be printed on the top side of the backing layer (i.e. second side of the medical dressing) or on the bottom side of the backing layer.

In order to prevent the machine-readable code from becoming chafed and damaged during use, the printed machine-readable code may be arranged on the bottom side of the backing layer. In such embodiments, the backing layer is transparent or translucent such that the machine-readable code is visibly observable on the second side of the medical dressing.

The medical dressing is defined by peripheral edges, and wherein at least a portion of the peripheral edges may be rounded or curved.

For example, the medical dressing may have a substantially square or rectangular shape, but the corners of the medical dressing may be rounded or curved. Alternatively, the medical dressing may be oval, circular or have an "irregular" shape with a plurality of protruding portions (to fit with e.g. the heel, elbow or sacrum of the patient).

The curvilinear shaped printed marking combined with curved or rounded peripheral edges yields an improved visual consistency (compared to the sharp and straight edges of a strictly square-shaped code).

In exemplary embodiments, the medical dressing may comprise a backing layer, an adhesive skin-contact layer, and an absorbent pad arranged between the backing layer and the adhesive skin-contact layer, wherein the absorbent pad is defined by pad edges; the backing layer and the adhesive skin-contact layer being arranged to extend beyond the pad edges to form a border portion surrounding the absorbent pad.

The at least one printed marking may be arranged in the border portion or in an area of the medical dressing overlying the absorbent pad.

In embodiments where the printed marking is arranged in an area of the medical dressing overlying the absorbent pad, the at least one printed marking is preferably arranged adjacent one of the pad edges.

For example, the printed marking(s) may be arranged adjacent a corner of the absorbent pad. This may be beneficial to facilitate scanning of the machine-readable code.

In exemplary embodiments, the machine-readable code may be a QR code, a bar code, a data matrix code or a shot code.

The machine-readable code may encode usage-specific and/or technical information connected to the medical dressing and/or patient-specific information.

For example, the machine-readable code may encode a link to instructions on how the medical dressing should be used, applied etc. Alternatively, the machine-readable code may encode information about the batch number, production lot number, and/or country indication. Such information may be beneficial for regulatory purposes, anti-counterfeiting and potential product recall purposes and enables authentication of the product. Accordingly, each dressing may be tracked and traced, e.g. back to the earliest steps of manufacturing.

The country indication may be useful for preventing illegal imports, i.e. branded products being imported into a market and sold there without the trademark owner's consent in that market or country.

It is also conceivable that the machine-readable code encodes patient-specific information, e.g. a link to a patient's database or journal, which may contain health care data of the patient.

Further features of, and advantages with, the present disclosure will become apparent when studying the appended claims and the following description. The skilled addressee realizes that different features of the present disclosure may be combined to create embodiments other than those described in the following, without departing from the scope of the present disclosure.

### BRIEF DESCRIPTION OF DRAWINGS

The various aspects of the present disclosure, including its particular features and advantages, will be readily understood from the following detailed description and the accompanying drawings, in which:
Figure 1 schematically illustrates the problem of misalignment associated with square-shaped machine-readable codes.
Figure 2a illustrates a substantially square-shaped medical dressing comprising a circular printed marking comprising a machine-readable code and being arranged in the border portion in accordance with an exemplary embodiment of the present disclosure.
Figure 2b illustrates the circular printed marking of figure 2a, wherein the printed marking comprises a square-shaped machine-readable code surrounded by a non-functional pattern.
Figure 3 illustrates a medical dressing adapted for application onto the heel and comprising a circular printed marking comprising a machine-readable code being arranged above the absorbent pad in accordance with an exemplary embodiment of the present disclosure.

### DETAILED DESCRIPTION

The present disclosure will now be described more fully hereinafter with reference to the accompanying drawings, in which currently preferred embodiments of the present disclosure are shown. The present disclosure may, however, be embodied in many different forms and should not be construed as limited to the embodiments set forth herein; rather, these embodiments are provided for thoroughness and completeness, and fully convey the scope of the present disclosure to the skilled person. Like references refer to like elements throughout.

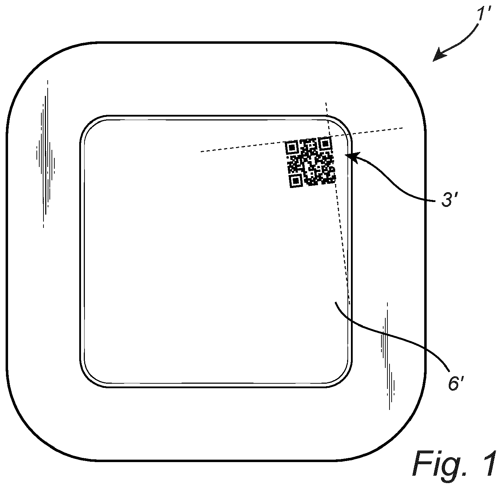

Figure 1 schematically illustrates the problems associated with square-shaped machine-readable codes.

As illustrated in figure 1, the square-shaped machine-readable code 3' is arranged in a skewed, i.e. "offset" manner with respect to the dressing edges, as well as the wound pad edges. In figure 1, the wound pad is denoted 6'.

The overall visual appearance of the medical dressing is significantly impaired. The "professional" impression associated with a sophisticated medical dressing is compromised. Furthermore, the machine-readable code 3' may become more difficult to scan and read by an electronic device.

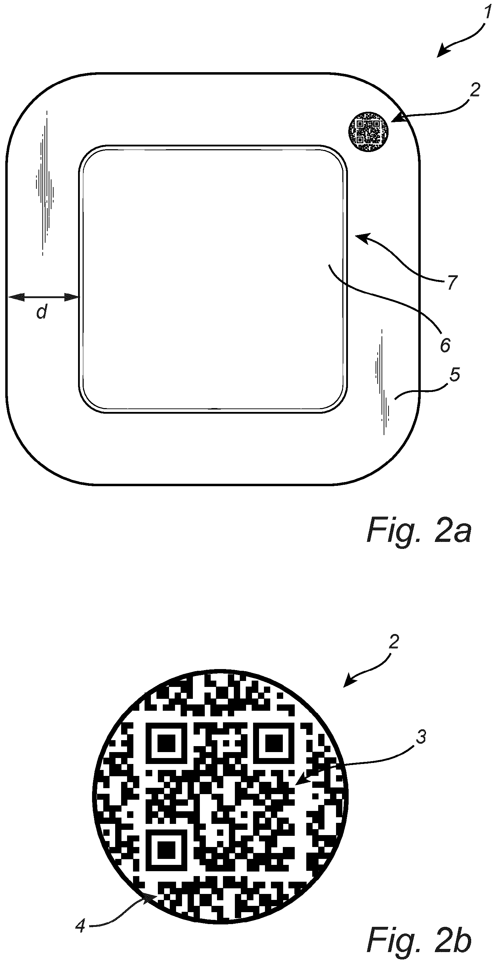

Figure 2a illustrates a medical dressing according to an exemplary embodiment of the present disclosure.

The medical dressing 1 has a first side facing the skin or wound of a patient during use, and an opposite, second side, wherein the medical dressing 1 comprises at least one printed marking 2 being visible on the second side of the medical dressing, characterized in that the printed marking 2 has a curvilinear shape wherein the printed marking 2 comprises a machine-readable code 3.

As used herein, the term "curvilinear shape" means a shape consisting of or being bounded by curved lines. Curvilinear shapes include, but are not limited to, arc shapes, elliptical, circular, and oval shapes.

The term "machine-readable code" is a code configured to store information in a format that machines, e.g. electronic devices, can quickly and accurately decode. Examples of machine-readable codes include QR codes, barcodes, data matrix codes, and shot codes.

The information encoded by the machine-readable code may be usage-specific and/or technical information connected to the medical dressing, or patient-specific information.

The machine-readable code may e.g. encode a link to usage-specific and/or technical information connected to the medical dressing and/or a link to patient-specific information.

As used herein the term "usage-specific information" means data, instructions, guidelines or specifications that pertain to the proper use, functionality and handling of the medical dressing. The information is intended to aid healthcare providers and patients in an effective and safe use of the medical dressing. Usage-specific information may include instructions for application and removal of the medical dressing, indications for use, precautions, guidance on storage conditions, handling procedures and disposal methods.

The term "technical information" may include details about the composition, materials, dimensions, and manufacturing process of the medical dressing. This may also include data sheets, technical specifications, performance characteristics and compliance with regulatory standards. For example, the technical information may include information about the batch number, production lot number, and/or country indication, which may be useful for e.g. product recall, regulatory and/or counterfeiting purposes.

As used herein, the term "patient-specific information" means information or data that may be unique to an individual patient. Such information may include personal medical data, such as information about the patient's name, age, medical history, allergies, specific wound characteristics (e.g. wound type, size, location, healing stage etc.). Patient-specific information may also include treatment plans, e.g. dressing change schedules, medication or topical treatments applied in conjunction with the medical dressing, and potential progress notes (e.g. observations of wound healing, adverse reactions etc.) from medical personnel. Furthermore, such information may also include a link to the patient's journal or health records.

The machine-readable code may have a surface area in the range of from in the range of from 80 to 500 mm², preferably from 100 to 300 mm².

This is to facilitate scanning and to secure readability of the machine-readable code when scanned by an electronic device, e.g. a mobile device.

In exemplary embodiment, the at least one printed marking 2 consists of a circular or an oval machine-readable code.

As mentioned hereinbefore, a circular shape is beneficial to overcome problems of misalignment and yielding a cohesive and visually appealing impression.

A circular code has a rotational symmetry. Hence, the circular code looks the same from multiple orientations.

An example of a circular machine-readable code is a shot code.

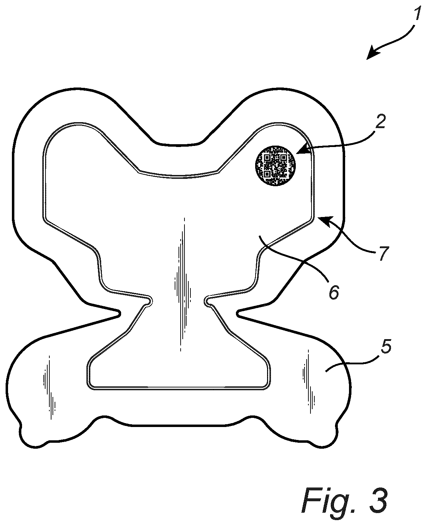

In figures 2a-b and 3, the at least one printed marking 2 comprises a functional pattern and a non-functional pattern 4, wherein the machine-readable code 3 forms the functional pattern, and wherein the non-functional pattern 4 forms the remaining part of the printed marking 2.

As used herein, the term "non-functional pattern" means a pattern of elements that do not encode any data or information. The non-functional pattern may comprise a plurality of elements selected from, but not limited to, squares, dots, lines etc. The visual appearance of the non-functional pattern resembles the visual appearance of the functional pattern. The non-functional pattern is configured to resemble the machine-readable code. The non-functional pattern is distinguished from the functional pattern, which is formed by the machine-readable code that encodes data and information.

As best illustrated in figure 2b, the functional pattern, i.e. the machine-readable code 3 may be arranged centrally in the printed marking 2.

The printed marking 2 has a circular shape, and the machine-readable code 3 is square-shaped.

The non-functional pattern 4 surrounds the square-shaped machine-readable code 3. A cohesive, circular printed marking 2 is thereby provided. When the printed marking 2 is arranged in an upper corner of the pad, as illustrated in figure 2a, the circular shape adapts to the rounded edges of the pad. The visual and "professional" appearance of the medical dressing is significantly improved (compared to the medical dressing illustrated in figure 1).

The maximum lateral extension of the printed marking 2 may be from 9 to 25 mm, e.g from 10 to 15 mm.

The maximum longitudinal extension of the printed marking 2 may be from 9 to 25 mm, e.g from 10 to 15 mm.

When the printed marking 2 has a circular shape (as in figures 2a-b and 3), the maximum lateral (and longitudinal) extension corresponds to the diameter of the printed marking 2.

The medical dressing 1 may comprise at least a backing layer 5 and an adhesive skin-contact layer (not shown), wherein the at least one printed marking 2 is printed on the backing layer 5.

In the context of the present disclosure, the "backing layer" is the top layer of the medical dressing, i.e. the outermost layer of the medical dressing. The backing layer is a continuous layer. In other words, the backing layer is void of perforations and incisions. The backing layer protects the layers of the medical dressing from potential contaminants.

The backing layer is desirably a thin and pliable layer such that it can stretch and conform to the movement of a wearer. The backing layer may be a polymeric film. Suitably, the backing layer comprises a polyurethane film. The backing layer may also be a laminate of a nonwoven layer and at least one polyurethane film. The thickness of the backing layer may be in the range of from 15 to 50 µm, preferably from 20 to 40 µm.

The backing layer 5 has a top side and a bottom side. The top side of the backing layer corresponds to the second side of the medical dressing, as mentioned hereinbefore.

The printed marking 2 may be arranged, i.e. printed on the top side or on the bottom side of the backing layer 5. To protect the printed marking from becoming damaged or chafed during use, the printed marking may be arranged on the bottom side of the backing layer.

The printed marking 2 is visibly observable on the second side of the medical dressing. Hence, in embodiments where the printed marking is printed on the bottom side of the backing layer (or on an underlying layer), the backing layer is transparent or translucent.

The printed marking comprising the machine-readable code may be formed by a colored ink. Any colored ink may be used. The ink may be water based or solvent based, i.e. the ink may comprise an organic solvent, such as an alcohol, ester etc. that can dissolve the pigment, resin and potentially other additives. For example, a flexographic ink may be used. The printed marking comprising the machine-readable code may be printed by means of any conventional printing technique known in the art, including, but not limited to a gravure printing, a flexographic printing, an offset printing, thermal transfer printing, an ink jet printing and the like.

The "adhesive skin-contact layer" of the medical dressing is configured to detachably adhere the medical dressing to a dermal surface. In other words, the adhesive skin-contact layer is configured to contact the skin or the wound of a wearer. This layer may also be referred to as a "wound contact layer".

The adhesive skin-contact layer has a top side facing the backing layer 5, and an opposing skin-contact side. The skin-contact side corresponds to the first side of the medical dressing described hereinbefore.

In some embodiments, the printed marking 2 may be arranged on the top side of the adhesive-skin contact layer.

The adhesive-skin contact layer may comprise a polymeric film and an adhesive silicone gel; the silicone gel being arranged to contact the skin of a wearer during use. Accordingly, the silicone gel forms first, skin-contact side of the medical dressing.

An adhesive skin-contact layer comprising a silicone gel is skin-friendly and easy to remove without causing trauma. It is sufficiently adherent to skin such that the medical dressing stays in place but is configured to maintain its adherence with repeated removal and re-application.

Examples of suitable silicone gels for use in the adhesive skin-contact layer include the two component RTV systems, such as Q72218 (Dow Corning), and SilGel 612 (Wacker Chemie AG), as well as NuSil silicone elastomers. In embodiments of the invention the adhesive may comprise a soft silicone gel having a softness (penetration) of from 8 to 22 mm, e.g. from 12 to 17 mm, as measured by a method based on ASTM D 937 and DIN 51580, the method being described in European Patent Application No 14194054.4. The thickness of the adhesive skin-contact layer is typically at least 20 µm. The thickness of the adhesive skin-contact layer may be from 50 to 200 µm.

The polymeric film may be a breathable film and may comprise e.g. polyethylene, polyamide, polyester or polyurethane. Preferably, the polymer-based film comprises polyurethane. The thickness of the polymeric film may be from 15 to 100 µm, e.g. from 20 to 80 µm, preferably from 20 to 60 µm.

The adhesive skin-contact layer may be translucent or transparent to improve the visibility of the printed marking.

Hence, the printed marking 2 may be visibly observable when viewed from the bottom-side i.e. first, skin-contact side of the medical dressing.

The medical dressing is defined by peripheral edges, and wherein at least a portion of the peripheral edges may be rounded or curved.

In the medical dressing illustrated in figure 2a, the corners of the square-shaped dressing are curved. The medical dressing illustrated in figure 3 is a heel dressing comprising a plurality of protruding portions. The medical dressing in figure 3 comprises several rounded or curved portions.

Rounded or curved corners and peripheral edges secure that the medical dressing conforms better to the contour of the body and may improve the stay-on ability of the medical dressing onto the skin.

A curvilinear shaped printed marking combined with curved or rounded dressing edges improves the overall visual impression of the medical dressing.

In each of the medical dressings illustrated in figure 2a and 3, the medical dressing further comprises an absorbent pad 6.

Hence, the medical dressing may comprise a backing layer 5, an adhesive skin-contact layer (not shown), and an absorbent pad 6 arranged between the backing layer 5 and the adhesive skin-contact layer, wherein the absorbent pad 6 is defined by pad edges; the backing layer 5 and the adhesive skin-contact layer being arranged to extend beyond the pad edges to form a border portion 7 surrounding the absorbent pad 6.

As illustrated in figures 2a and 3, at least a portion of the pad edges may be rounded or curved.

In the context of the present disclosure, the "absorbent pad" may comprise one or a plurality of pad-forming layers.

The absorbent pad may e.g. comprise an absorbent polyurethane foam layer.

The absorbent pad may further comprise a superabsorbent layer, i.e. a layer comprising a superabsorbent material. The superabsorbent material may be superabsorbent polymer (SAP) particles or superabsorbent fibres (SAF). The superabsorbent material is capable of handling large amounts of wound exudate.

The absorbent pad may also comprise a liquid distribution layer. The liquid distribution layer may comprise any material having the ability to distribute the exudate in an efficient manner. For example, the liquid distribution layer may comprise a nonwoven material.

A layered pad construction prevents accumulation of body liquids close to the skin and improves the overall liquid handling of the medical dressing.

The backing layer may be attached to the adhesive skin-contact layer in the area of the border portion. For example, the backing layer may be attached to the adhesive skin-contact layer by an adhesive, e.g. a polyacrylate adhesive. Heat lamination of the backing layer and the adhesive skin-contact layer is also conceivable.

The backing layer and the adhesive skin-contact layer are co-extensive, i.e. they have the same length and width. This is the case also in embodiments where the medical dressing is void of an absorbent pad.

The size of the absorbent pad 6 is smaller than the size of the backing layer 5 and the adhesive skin-contact layer.

The maximum width, i.e. maximum lateral (x) extension of the dressing may be from 35 to 250 mm.

The maximum length, i.e. maximum longitudinal (y) extension of the dressing may be from 50 to 450 mm.

The medical dressing typically further comprises a release liner detachably attached to the adhesive skin-contact layer. The release liner may comprise one or a plurality of release liner portion and is configured to cover the entire surface of the adhesive skin-contact layer.

The border portion 7 surrounds the absorbent pad. In other words, the border portion extends a distance, d, beyond each peripheral edge of the absorbent pad 6 (see figure 2a).

The distance, d, may be the same or it may differ. For example, if the medical dressing has a more "complex" shape, as is the case with the heel dressing illustrated in figure 3, some parts of the medical dressing may have a larger border portion. Accordingly, the distance, d, may vary along the peripheral edges of the medical dressing. Alternatively, the distance, d, is the same around the whole periphery of the absorbent pad.

In order to secure a firm attachment to the skin and to enable printing, the border portion may have a width (distance, d) of at least 10 mm, e.g. from 10 to 50 mm.

A smaller sized dressing may have a smaller border portion than a larger sized dressing.

The adhesive skin-contact layer may comprise a plurality of perforations in the area underlying the absorbent pad but may be void of perforations in the area forming the border portion.

The area of the adhesive-skin contact layer forming the border portion is preferably non-perforated. Hence, the entire border portion is void of perforations. This is beneficial to improve the adhesion to the skin in the border of the medical dressing such that the stay-on ability of the medical dressing is improved.

The perforations in the adhesive skin-contact layer arranged underneath the absorbent pad improve the absorption of wound exudate and liquid handling of the medical dressing and are therefore arranged in the area where absorption takes place.

The perforations may be of various shapes and sizes. Preferably, the perforations are arranged in a predetermined, regular pattern. The perforations may have a diameter of from 0.5 mm to 10 mm, e.g. from 1 to 5 mm, preferably from 1 to 2 mm.

The at least one printed marking 2 may be arranged in the border portion of the medical dressing (as illustrated in figure 2a) or in an area of the medical dressing overlying the absorbent pad (as illustrated in figure 3).

In embodiments where the at least one printed marking 2 is arranged in an area of the medical dressing overlying the absorbent pad 6, the printed marking 2 is preferably arranged adjacent one of the pad edges. Preferably the printed marking 2 is arranged in a corner of the pad (as illustrated in figure 3).

The medical dressing of the present disclosure may comprise a plurality of printed markings, i.e. a plurality of machine-readable codes, as described hereinbefore. This may be beneficial to enable scanning of the machine-readable codes from multiple orientations.

Furthermore, the printed marking described hereinbefore may form part of a logotype arranged on the medical dressing.

Terms, definitions and embodiments of all aspects of the present disclosure apply mutatis mutandis to the other aspects of the present disclosure.

Even though the present disclosure has been described with reference to specific exemplifying embodiments thereof, many different alterations, modifications and the like will become apparent for those skilled in the art.

Variations to the disclosed embodiments can be understood and effected by the skilled addressee in practicing the present disclosure, from a study of the drawings, the disclosure, and the appended claims. Furthermore, in the claims, the word "comprising" does not exclude other elements or steps, and the indefinite article "a" or "an" does not exclude a plurality.

## Claims

1. A medical dressing (1) having a first side facing the skin or wound of a patient during use, and an opposite, second side, wherein said medical dressing (1) comprises at least one printed marking (2) being visible on said second side of said medical dressing, **characterized in that** said printed marking (2) has a curvilinear shape and **in that** said printed marking (2) comprises a machine-readable code (3).

2. The medical dressing (1) according to claim 1, wherein said at least one printed marking (2) consists of a circular or an oval machine-readable code.

3. The medical dressing (1) according to claim 1, wherein said at least one printed marking (2) comprises a functional pattern and a non-functional pattern (4), wherein said machine-readable code (3) forms said functional pattern, and wherein said non-functional pattern (4) forms the remaining part of said printed marking (2).

4. The medical dressing (1) according to claim 3, wherein said functional pattern is arranged centrally in said printed marking (2).

5. The medical dressing (1) according to any one of the preceding claims, wherein said printed marking (2) has a circular shape.

6. The medical dressing (1) according to any one of claims 1, 3-5, wherein said machine-readable code (3) is square-shaped.

7. The medical dressing (1) according to any one of claims 3-6, wherein the visual appearance of said non-functional pattern (4) resembles the visual appearance of said functional pattern.

8. The medical dressing (1) according to any one of claims 3-7, wherein said non-functional pattern (4) comprises a plurality of squares, dots or lines configured to resemble said machine-readable code (3).

9. The medical dressing (1) according to any one of the preceding claims, wherein the surface area of said machine-readable code (3) is from 80 to 500 mm², preferably from 100 to 300 mm².

10. The medical dressing (1) according to any one of the preceding claims, wherein said medical dressing (1) comprises at least a backing layer (5) and an adhesive skin-contact layer, wherein said at least one printed marking (2) is printed on said backing layer (5).

11. The medical dressing (1) according to any one of the preceding claims, wherein said medical dressing is defined by peripheral edges, and wherein at least a portion of said peripheral edges is rounded or curved.

12. The medical dressing (1) according to any one of the preceding claims, wherein said medical dressing (1) comprises a backing layer (5), an adhesive skin-contact layer, and an absorbent pad (6) arranged between said backing layer (5) and said adhesive skin-contact layer, wherein said absorbent pad (6) is defined by pad edges; said backing layer (5) and said adhesive skin-contact layer being arranged to extend beyond said pad edges to form a border portion (7) surrounding said absorbent pad (6).

13. The medical dressing (1) according to claim 12, wherein said at least one printed marking (2) is arranged in said border portion (7) or in an area of said dressing overlying said absorbent pad (6), preferably adjacent one of said pad edges.

14. The medical dressing (1) according to any one of the preceding claims, wherein said machine-readable code is a QR code, a bar code, a data matrix code or a shot code.

15. The medical dressing (1) according to any one of the preceding claims, wherein said machine-readable code encodes usage-specific and/or technical information connected to the medical dressing and/or patient-specific information.
